# EUROPEAN PATENT SPECIFICATION

(11) **EP 4 183 857 B1**
(45) Date of publication and mention of the grant of the patent: **29.01.2025**
(21) Application number: 22208376.8
(22) Date of filing: 18.11.2022
(51) Int. Cl.: C10M 105/30, B21C 23/32

(54) **USE OF A LUBRICANT RELEASE AGENT COMPOSITION**
VERWENDUNG EINER SCHMIERMITTEL-TRENNMITTEL-ZUSAMMENSETZUNG
UTILISATION D'UNE COMPOSITION D'AGENT DE DÉMOULAGE DE LUBRIFIANT

(30) Priority: 23.11.2021 IT 202100029573
(43) Date of publication of application: 24.05.2023
(73) Proprietor: Baraldi S.r.l., 40024 Castel San Pietro Terme (Bologna) (IT)
(72) Inventor: RAONE, Cosimo, Castel Guelfo (Bologna) (IT)
(74) Representative: Delbarba, Andrea

(56) References cited:
- US-A- 4 834 891
- US-A1- 2018 251 699

## Description

This invention relates to the use of a lubricant release agent composition in a process for extruding a metal.

### BACKGROUND ART

The extrusion of metal materials is a process by which, by means of a plastic deformation, parts with a constant cross-section are obtained, for example plates, pipes, profiled sections and bars.

The extrusion process is used in industrial production for metal materials such as aluminium, copper, lead and steel. The material which must be processed and transformed is usually in the pasty state, in the form of pallets or powder and is forced by compression into a mould, in order to obtain the desired product, in practice an outer shape to be subsequently used.

The moulding is performed by compression of the metal material, in the form of a billet, through a die designed ad hoc to give the desired shape to the material being processed. It is essential that the metal is in a pasty melting or softening state, so that the plastic formation passage can occur. Once the metal material has been acquired, for example aluminium, this is brought to the heating oven, which has a temperature of between 440 and 500°C; the product is then pressed with a piston against a steel die and the extruded material moves along a roller surface, where it is cooled. The final section of the billet, corresponding to some tens of mm (near the piston), since it is as a potential source of imperfections for the profiles and impurities, is not extruded, but cut at the end of the cycle, by means of an automatic shearing device and then recovered by remelting (billet bottom). Due to the high temperature of the billet, the billet metal tends to attach to the piston head of the press and to remain welded to said head and it can lock the piston causing damage to the press.

Various solutions have been introduced to prevent the billet from remaining adhered to the piston head, for example, grease and oil and/or wax-based pastes with or without adding solid pigments such as graphite, aluminium and others are used. However, this type of product requires a manual application with a pad or brush. Due to the high temperature, these products can result in dripping (loss and soiling of the work environment) and invariably give rise to the development of rather dense fumes and vapours. Moreover, said products are combustible and can catch fire.

A second solution relates to the use of a layer of carbon black which, thanks to a short combustion, deposits on the treated surface. However, due to the combustion the use of said product is dangerous and may be harmful if inhaled.

Boron nitride is a synthetic ceramic material which at the working temperatures does not undergo alterations. This product is particularly effective if deposited in such a way as to perfectly cover the entire treated surface. As it is not, in fact, able to migrate on the surface, any areas left uncovered give rise to metallization and gluing between the press heel and the billet. Moreover, the very light powder spreads into the environment and can be inhaled.

Moreover, water-based products, when sprayed on very hot surfaces (> 200°C), are subject to the calefaction phenomenon (or Leidenfrost effect) whereby a vapour barrier forms instantly between sprayed droplets and metal, which prevents direct contact of liquid-metal and the film deposition. The application of these materials inevitably results in incomplete and nonuniform dripping and film, which means that these products fall in a category which is not widespread, although it is still used.

US4834891 discloses a lubricant composition comprising an alkali metal salt of phthalic acid in a concentration of 60% to 90% wt together with a glass composition including P₂O₅, M₂O and B₂O₃.

US2018/251699 discloses a dry lubricant composition in powder form especially useful for cold forming of aluminium cans.

For this reason, there is a strongly felt need to overcome the above-mentioned technical problem, avoiding the above-mentioned drawbacks.

### SUMMARY OF THE INVENTION

The invention relates to the use of a lubricant release agent composition in a process for extruding metals. Said composition consists of sodium phthalate selected from among monosodium phthalate or disodium phthalate, the disodium phtalate is selected from among disodium orthophthalate, disodium isophtalate and disodium terephthalate.

The composition is preferably in the form of a powder with a grain size of between 5 and 120 µm. According to an embodiment, the composition is applied on at least one surface 101 of a head 100 of a piston 10 of an extrusion press 1 and/or on at least one surface 201 of a metal billet 20. Preferably, the composition is applied before the start of the extrusion process in order to facilitate the detachment of the at least one surface 101 of the head 101 of the piston 10 from the billet 20 at the end of the extrusion process.

### BRIEF DESCRIPTION OF THE DRAWING

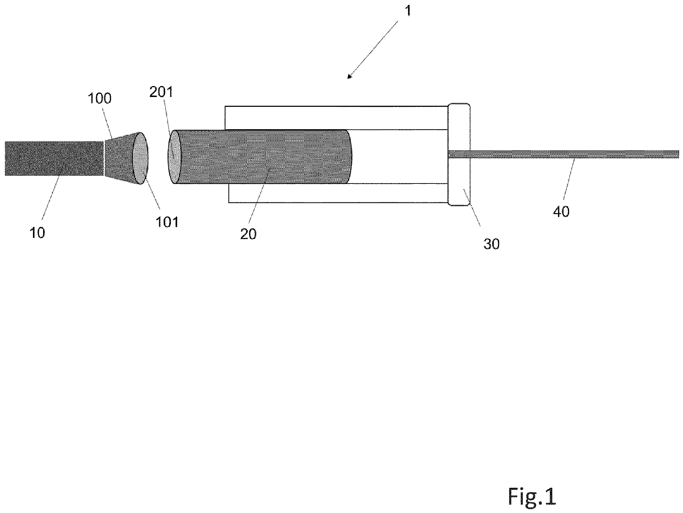

Figure 1 shows a diagram of a press 1 for the extrusion of a metal.

### DETAILED DESCRIPTION OF THE INVENTION

The invention relates to the use of a lubricant release agent composition in a process for extruding metals. Said composition consists of sodium phthalate selected from among monosodium phthalate or disodium phthalate, the disodium phtalate is selected from among disodium orthophthalate, disodium isophtalate and disodium terephthalate.

According to an embodiment, the composition is in the form of powder. Preferably, the composition is in the form of powder with a grain size of between 10 and 150 µm, more preferably between 5 and 120 µm.

According to the disclosure, the composition comprises at least one solid pigment, preferably said at least one solid pigment is selected from among: talc, mica, boron or graphite nitride, or other minerals.

According to a preferred embodiment, the composition consists of disodium orthophthalate.

According to an embodiment, the composition is applied on at least one surface 101 of a head 100 of a piston 10 of an extrusion press 1 and/or on at least one surface 201 of a non-ferrous metal billet 20. Preferably, the composition is applied before the start of the extrusion process in order to facilitate the detachment of the surface 101 of the head 101 of the piston 10 from the billet 20 at the end of the extrusion process. Preferably, the extrusion process is a process for extruding a non-ferrous metal made of aluminium or copper or alloys thereof with other metals.

According to an embodiment, the composition is applied on the at least one surface 101 of the head 100 of the piston 10 of the press 1 and/or on the at least one surface 201 of the metal billet 20 with systems and methods known to an expert in the trade. Preferably, the composition is applied by means of a spray, preferably using an electrostatic type spray system.

The Applicant has surprisingly found that the composition according to the invention is able to prevent the metal of the billet 20 from sticking to the head 100 of the piston 10 or remaining welded thereto and, therefore, the piston 10 remaining locked, or creating damage due to breakage of the tools of the press 1 when retracting. The composition can be therefore be defined as a lubricant release agent.

The composition according to the invention has a melting point of between 400 and 450 °C, slightly less than or equal to the extrusion process temperatures. For this reason, after its application on the at least one surface 101 of the head 100 of the piston 10 of the press 1 and/or on the at least one surface 201 of the metal billet 20 it starts to melt thanks to the high temperature of the billet 20 or the head 100 of the piston 10 and to spread spontaneously on the treated surface. The subsequent compression between the head 100 of the piston 10 and the billet 20 leads to the complete stretching of a film which covers the entire surface in contact forming a complete and continuous barrier film.

Moreover, the use of the composition according to invention reduces the consumption with respect to boron nitride, since the product does not create static accumulations of powder, but always covers the surface regardless of how small the quantity applied.

In order to reduce the consumption of the costly boron nitride as much as possible, a grain size of between 2 and 30 microns is used with an average value of around 10 microns, such as to guarantee a covering capacity of 5-15 m²/g. The composition according to the invention comprises sodium or potassium phthalate with a much greater grain size, of between 20 and 100 microns, due to the melting property which increases the covering capacity. This means that during the spray dispensing the boron nitride tends to spread in the surrounding space more intensely and rapidly compared to sodium or potassium phthalate, which, on the other hand, reaches the surface to be treated due to the electrostatic charge, without further spreading into the air. In this way, the composition spreads very little outside the treated surfaces, avoiding affecting the press 1, the peripherals of the press and the surrounding environments. Above all, the extent of the amount inhaled by the operators present in the proximity of the machine is lower. Lastly, this reduces defects due to inclusions and streaks of the profiled sections 40 obtained with the composition according to the invention.

## Claims

1. A use in a process for extruding non-ferrous metals of a lubricant release agent composition consisting of sodium phthalate selected from among monosodium phthalate or disodium phthalate, wherein the disodium phtalate is selected from among disodium orthophthalate, disodium isophtalate and disodium terephthalate.

2. The use according to claim 1, wherein the composition is the form of a powder.

3. The use according to claim 1 or 2, wherein the disodium phthalate is disodium orthophthalate.

4. The use according to claim 2 or 3, wherein the composition has a grain size of between 10 and 150 µm.

5. The use according to claim 4, wherein the composition has a grain size of between 5 and 120 µm.

6. The use according to any one of claims 1 to 5, wherein the composition is applied on at least one surface (101) of a head (100) of a piston (10) of an extrusion press (1) and/or on at least one surface (201) of a billet (20) of a non-ferrous metal.

## Patentansprüche

1. Verwendung einer Schmiermittel-Trennmittel-Zusammensetzung, bestehend aus Natriumphthalat, ausgewählt aus Mononatriumphthalat oder Dinatriumphthalat, wobei das Dinatriumphthalat ausgewählt ist aus Dinatrium-Orthophtalat, Dinatrium-Isophthalat und Dinatrium-Terephthalat, in einem Prozess zum Extrudieren von NE-Metallen.

2. Verwendung nach Anspruch 1, wobei die Zusammensetzung die Form eines Pulvers aufweist.

3. Verwendung nach Anspruch 1 oder 2, wobei das Dinatriumphthalat Dinatrium-Orthophthalat ist.

4. Verwendung nach Anspruch 2 oder 3, wobei die Zusammensetzung eine Korngröße zwischen 10 und 150 µm aufweist.

5. Verwendung nach Anspruch 4, wobei die Zusammensetzung eine Korngröße zwischen 5 und 120 µm aufweist.

6. Verwendung nach einem der Ansprüche 1 bis 5, wobei die Zusammensetzung auf mindestens eine Oberfläche (101) eines Kopfs (100) eines Kolbens (10) einer Extrusionspresse (1) und/oder auf mindestens eine Oberfläche (201) eines Rohlings (20) aus einem NE-Metall aufgetragen wird.

## Revendications

1. Utilisation, dans un procédé d'extrusion de métaux non ferreux, d'une composition d'agent de démoulage de lubrifiant constituée de phtalate de sodium choisi parmi le phtalate monosodique ou le phtalate disodique, le phtalate disodique étant choisi parmi l'orthophtalate disodique, l'isophtalate disodique et le téréphtalate disodique.

2. Utilisation selon la revendication 1, dans laquelle la composition se présente sous la forme d'une poudre.

3. Utilisation selon la revendication 1 ou 2, dans laquelle le phtalate disodique est l'orthophtalate disodique.

4. Utilisation selon la revendication 2 ou 3, dans laquelle la composition a une granulométrie comprise entre 10 et 150 µm.

5. Utilisation selon la revendication 4, dans laquelle la composition a une granulométrie comprise entre 5 et 120 µm.

6. Utilisation selon l'une quelconque des revendications 1 à 5, dans laquelle la composition est appliquée sur au moins une surface (101) d'une tête (100) d'un piston (10) d'une presse à filer (1) et/ou sur au moins une surface (201) d'une billette (20) d'un métal non ferreux.
